# EUROPÄISCHE PATENTSCHRIFT

(11) **EP 1 206 253 B1**
(45) Veröffentlichungstag und Bekanntmachung des Hinweises auf die Patenterteilung: **13.10.2004**
(21) Anmeldenummer: 00964016.0
(22) Anmeldetag: 14.08.2000
(51) Int. Cl.: A61K 9/51, A61K 47/48

(54) **FESTE PHARMAZEUTISCHE ZUBEREITUNGEN MIT NANOSOLS AUS CHITOSAN, SOWIE DEREN HERSTELLUNG**
PHARMACEUTICAL PREPARATIONS CONTAINING NANOSOLS OF CHITOSAN, AND THEIR PREPARATION
PREPARATIONS PHARMACEUTIQUES CONTENANT DES NANOSOLS DE CHITOSANE, ET METHODE DE PREPARATION

(30) Priorität: 27.08.1999 DE 19940794
(43) Veröffentlichungstag der Anmeldung: 22.05.2002
(73) Patentinhaber: LTS Lohmann Therapie-Systeme AG, 56626 Andernach (DE)
(72) Erfinder: HOFFMANN, Hans-Rainer, 56566 Neuwied (DE); ASMUSSEN, Bodo, 56170 Bendorf-Sayn (DE)
(74) Vertreter: Flaccus, Rolf-Dieter, Dr.
(86) Internationale Anmeldenummer: PCT/EP2000/007904
(87) Internationale Veröffentlichungsnummer: WO 2001/015669

(56) Entgegenhaltungen:
- EP-A- 0 860 166
- WO-A-97/47323
- WO-A-99/36090
- DE-A- 4 140 195
- DE-A- 19 845 246
- US-A- 5 474 989
- TOKUMITSU H ET AL: "CHITOSAN-GADOPENTETIC ACID COMPLEX NANOPARTICLES FOR GADOLINIUM NEUTRON-CAPTURE THERAPY OF CANCER: PREPARATION BY NOVEL EMULSION-DROPLET COALESCENCE TECHNIQUE AND CHARACTERIZATION" PHARMACEUTICAL RESEARCH,NEW YORK, NY,US, Bd. 16, Nr. 12, Dezember 1999 (1999-12), Seiten 1830-1835, XP000951445 ISSN: 0724-8741

## Beschreibung

Pharmazeutische Zubereitungen, in denen ein Wirkstoff an einen Träger gebunden vorliegt, sind dem Stand der Technik in großer Fülle bekannt. Die Bindung an den Träger kann dabei im weitesten Sinn rein mechanisch verstanden werden; im engeren Sinn jedoch wird die Fähigkeit von Trägersubstanzen ausgenutzt, spezielle chemische oder physikochemische Wechselwirkungen mit dem Wirkstoff oder den Wirkstoffen einzugehen.

Eine Kategorie derartiger Wechselwirkungen bilden die ionischen Anziehungskräfte, die natürlich nur dann ausgenutzt werden können, wenn Wirkstoff und Träger zumindest teilweise geladen vorliegen. In pharmazeutischen Zubereitungen werden ionische Bindungen zwischen Wirkstoffen und Trägern u. a. dazu ausgenutzt, schwerlösliche Wirkstoffe mit geringer Dissoziatioasneiguag in Wasser in ihrem geladenen und molekulardispersen Zustand zu konservieren und damit eine hohe Auflösegeschwindigkeit zu erzielen. Daneben werden Wirkstoffe an gegensinnig geladene Trägerpolymere, gebunden, um eine hohe Wirkstoffbeladung der Zubereitung zu ermöglichen; diese Formulierungstechnik wird z. B. bei Liposomenzubereitungen häufig angewandt. Eine weitere beschriebene Variante bilden die Zubereitungen, in denen durch die ionische Bindung an ein geladenes Polymer eine kontrollierte Wirkstoffreisetzung bewirkt werden soll. Ein Beispiel hierfür stellt das in Deutschland unter der Marke Codipront® vermarktete Hustensaft dar, in dem als Wirkstoff-Trägerkomplex eine an einen sauren lonentauscher gebundene Wirkstoffbase, Codein-Poly(styrol, divinylbenzol)sulfonat, enthalten ist.

Eine spezielle Form der an gegensinnig geladene Träger gebundenen Wirkstoffe stellen die sog. Nanosole mit Gelatine oder Kollagenhydrolysaten als Trägern, die von der Fa. Alfatec-Pharma GmbH in verschiedenen Patent- und Offenlegungsschriften beschrieben werden, so z. B. in den Dokumenten DE 41 40 195, DE 41 40 178 und DE 41 40 179. Dabei macht man sich zunutze, daß der angestrebte, isoionische Zustand mit Ladungsausgleich zwischen Träger und Wirkstoff bei der Verwendung von Gelatine oder Gelatinederivaten dank der zwitterionischen Natur derselben durch eine entsprechende pH-Einstellung- in der Zubereitung leicht erzielt werden kann. Es wird beschrieben, daß sich diese Nanosole vorteilhaft zur Herstellung von Arzneizubereitungen sowohl mit schneller als auch mit kontrollierter Wirkstoffreisetzung einsetzen lassen.

Allerdings haben diese Zubereitungen den Nachteil, daß die Bevölkerung seit einigen fahren hinsichtlich möglicher BSE-Infektionsgefahren verunsichert ist und z. B. Produkte, die Gelatine enthalten, zunehmend meidet. Daher besteht ein Bedarf für Zubereitungen ohne Gelatine oder Kollagenderivate, welche die gleichen Vorteile aufweisen wie z. B. die beschriebenen Nanosole auf Gelatinebasis.

Die WO 99/36090 betrifft eine pharmazeutische Zubereitung aus Chitosan-Partikeln und Nukleinsäuren, die durch Koazervation hergestellt wird, welche mittels Zugabe von 5 mM Natriumsulfat induziert wird.

In der WO 97/47323 wird ein Verfahren beschrieben, in dem einem Chitosanderivat ein Metallsalz zugesetzt wird, dessen Metall zu einem integralen Bestandteil der Chitosanpartikel werden kann.

In der EP-A-0 860 166 wird ein Verfahren beschrieben, in dem ein ionisches Vernetzungsmittel verwendet wird, um bioaktive Makromoleküle in die chitosanhaltigen Nanopartikel einzuarbeiten.

Tokumitsu H, et al., Pharmaceutical Research Vol.16, No.12, pp 1830-1835, 1999 beschreiben ein Verfahren zur Herstellung von Chilosan-Nanopartikeln, welche mit Gadopentensäure beladen sind.

Es ist daher Aufgabe der vorliegenden Erfindung, eine pharmazeutische Zubereitung ohne Gelatine oder dergleichen für geladene Wirkstoffe sowie eine Verfahren zu ihrer Herstellung und ihrer Verwendung bereitzustellen, in denen der Wirkstoff an einen gegensinnig geladenen Träger gebunden vorliegt.

Die Lösung dieser Aufgabe gelingt durch eine pharmazeutische Zubereitung nach Anspruch 4, eine Verfahren nach Anspruch 1, sowie die Verwendung nach Anspruch 8.

Überraschenderweise wurde gefunden, daß sich mit den erfindungemäßen Chitosanderivaten als Träger sog. Nanosole erzeugen lassen, in denen der Wirkstoff mit dem Träger im zumindest annähernd isoionischen Zustand stabilisiert vorliegt, und daß sich diese Nanosole zur Herstellung von Arzneimitteln bestens eignen.

Die erfindungsgemäße Zubereitung enthält nach Anspruch 4 mindestens einen pharmazeutischen Wirkstoff, der zumindest teilweise geladen vorliegt, der dadurch gekennzeichnet ist, daß er in Form eines Nanosalz vorliegt, wobei der Wirkstoff eine positive Ladung aufweist und an ein negativ geladenes oder zitterionisches, Saures Chitosanderivate gebunden ist.

Für die Definition eines Nanosols wird auf DE 41 40 195 verwiesen.

Als erfindungsgemäß negativ geladene oder zwitterionische, saure Chitosanderivate im Sinne dieser Erfindung gelten alle modifizierten und nichtmodifizierten Deacetylierungsprodukte des Chitins, die noch eine Polyglucosamin-Grundstru.ktur aufweisen. Die erfindungsgemäß geforderte, zum Wirkstoff gegensinnige Ladung bezieht sich auf die Nettoladung des eingesetzten Trägers. Es können demnach auch zum Wirkstoff gleichsinnige Ladungen im Chitosanderivat vorhanden sein, solange diese von den gegensinnigen Ladungen überkompensiert werden.

In einer Ausführungsform kann es sich bei einem solchen erfindungsgemäßen Chitosanderivat z. B. um ein zwitterionisches, partiell sulfatiertes Chitosan handeln.

Vorzugsweise liegt der Wirkstoff im Nanosol in einer kolloidalen oder nanopartikulären Verteilung, d. h. mit einer mittleren Teilchengröße von maximal etwa 500 - 1000 nm vor, soweit sich überhaupt eine Phasengrenze zwischen Wirkstoff und Trägerphase nachweisen läßt. Insbesondere schwerlösliche Wirkstoffe lassen sich auf diese Weise in pharmazeutische Zubereitungen einarbeiten, aus denen sie rasch freigesetzt werden können.

Die erfindungsgemäßen Zubereitungen werden in der Regel weitere Hilfsstoffe enthalten, die in der pharmazeutischen Technologie üblicherweise eingesetzt werden und dem Fachmann bekannt sind. Bei diesen Hilfsstoffen kann es sich beispielsweise um weitere polymere oder nichtpolymere Trägerstoffe handeln, aber auch um Stabilisatoren, Tenside, Zerfallsbeschleuniger, Antioxidantien, Farbstoffe, Pigmente, Aromen, Süßstoffe oder sonstige Geschmacksverbesserer, Bindemittel, Gleitmittel u. s. w. In einer bevorzugten Ausführungsform enthält die Zubereitung einen weiteren polymeren Trägerstoff. Dieser kann z. B. erforderlich sein, um die Beladbarkeit des Nanosols mit Wirkstoff zu erhöhen oder um die Freisetzungseigenschaften der Zubereitung zu modifizieren. Diesbezügliche Formulierungstechniken sind dem Fachmann ebenfalls bekannt.

Erfindungsgemäß werden die hier offenbarten pharmazeutisehen Zubereitungen zur Herstellung von Arzneimitteln oder Diagnostika verwendet. Eine bevorzugte Verwendung der Zubereitungen besteht in der Herstellung von Arzneimitteln, die als Kapseln, Tabletten, Pulver oder Granulate verabreicht werden oder im Sinne einer Instantzubereitung vor ihrer Verabreichung zunächst in Wasser oder einer anderen, geeigneten Flüssigkeit aufgelöst oder redispergiert werden.

In einer weiteren, bevorzugten Ausführung werden die Zubereitungen zur Herstellung von Arzneimitteln mit kontrollierter Wirkstoffreisetzung verwendet. Dazu müssen sie in der Regel weiter modifiziert, d. h. mit weiteren Hilfsstoffen gemischt oder von diesen umhüllt werden. Z. B. können Kapseln oder Tabletten, die eine erfindungsgemäße Zubereitung enthalten, mit einem Polymerfilm überzogen werden, welcher die Freisetzung des Wirkstoffs oder der Wirkstoffe kontrolliert. Diese und weitere Techniken zur Herstellung von Arzneiformen mit modifizierter oder kontrollierter Wirkstoffreisetzung sind dem Fachmann bekannt.

Eine erfindungsgemäße Zubereitung wird grundsätzlich in einem mehrschrittigen Verfahren hergestellt, welches bedarfsweise variiert oder durch weitere Schritte ergänzt werden kann. Zunächst wird unter Berücksichtigung der relativen Anzahl und Art der geladenen Gruppen des Wirkstoffs ein negativ geladenes oder zwitter-ionisches, Saures Chitosanderivat als Träger ausgewählt, welches durch die Art und relative Anzahl seiner geladenen Gruppen so auf den Wirkstoff abgestimmt ist, daß bei einem bestimmten pH-Wert ein isoionischer Zustand bzw. ein Laungsausgleich zwischen Wirkstoff und Träger erzielt wird. Dies ist in der Regel dann der Fall, wenn die Nettoladungen von Wirkstoff und Chitosanderivat gegensinnig sind und der errechnete isoionische Punkt in einem pH-Bereich liegt, welcher physiologisch akzeptabel und der Stabilität des Wirkstoffs nicht abträglich ist.

In einem weiteren Schritt wird aus dem Chitosanderivat und dem Wirkstoff eine kolloidale wäßrige Lösung hergestellt, welche aufgrund ihres Polymergehalts und der daraus resultierenden Viskosität ein Sol darstellt. Dabei ist es unerheblich, ob die Zugabe des Wirkstoffs nach oder vor dem Lösen des Chitosanderivats geschieht oder ob eine Lösung des Chitosanderivats und eine unabhängig hergestellte Lösung des Wirkstoffs miteinander vereinigt werden.

In einem weiteren Schritt wird der pH-Wert des wäßrigen Sols so eingestellt, daß ein isoionischer Zustand entsteht. Bei dieser pH-Verschiebung kann es zu einer Ausfällung des Wirkstoffs kommen, wobei es sich gezeigt hat, daß die Partikel in der Regel den kolloidalen oder nanopartikulären Größenordnungsbereich nicht überschreiten.

Das derart vorbereitete und auf einen isoionischen Zustand eingestellte Sol wird in einem weiteren Verfahrensschritt getrocknet. Hierzu können herkömmliche Trocknungsverfahren, vorzugsweise jedoch Trocknungsverfahren ohne mit nur geringer Anwendung von Wärme wie z. B. die Gefriertrocknung eingesetzt werden.

## Patentansprüche

1. Verfahren zur Herstellung einer festen pharmazeutischen Zubereitung mit mindestens einem zumindest teilweise geladenen Wirkstoff, der in Form eines Nanosols vorliegt, in welchem der Wirkstoff an ein gegensinnig geladenes Chitosanderivat gebunden ist, **dadurch gekennzeichnet, daß**
- ein wässriges Sol aus einem negativ geladenen oder zwitterionischen sauren Chitosanderivat und einem eine positive Ladung aufweisenden Wirkstoff hergestellt wird, indem der Wirkstoff vor oder nach dem Lösen des Chitosanderivats zugegeben wird oder eine Lösung des Chitosanderivats und eine unabhängig hergestellte Lösung des Wirkstoffs miteinander vereinigt werden, wobei das Chitosanderivat nach Art und relativer Anzahl seiner geladenen Gruppen und in Abstimmung mit der Art und relativen Anzahl der positiv geladenen Gruppen des Wirkstoffs so ausgewählt wird, daß bei einem bestimmten pH-Wert in der Zubereitung ein isoionischer Zustand oder Ladungsausgleich zwischen Wirkstoff und Träger erzielt wird,
- der pH-Wert des wässrigen Sols so eingestellt wird, daß ein isoionischer Zustand entsteht, wobei kolloidale oder nanoskalige Wirkstoffpartikel ausfallen können, und
- das derart eingestellte wässrige Sol getrocknet wird.

2. Verfahren nach einem der vorangehenden Ansprüche, **dadurch gekennzeichnet, daß** ein schwerlöslicher Wirkstoff verwendet wird.

3. Verfahren nach einem der vorangehenden Ansprüche, **dadurch gekennzeichnet, daß** neben dem Chitosanderivat ein weiterer polymerer Trägerstoff verwendet wird.

4. Feste pharmazeutische Zubereitung mit mindestens einem zumindest teilweise geladenen Wirkstoff, **dadurch gekennzeichnet, daß** der Wirkstoff in Form eines Nanosols vorliegt, wobei der wirkstoff eine positive Ladung aufweist und an ein negativ geladenes oder zwitterionisches, saures Chitosanderivat gebunden ist.

5. Feste pharmazeutische Zubereitung nach Anspruch 4, **dadurch gekennzeichnet, daß** der Wirkstoff im Nanosol kolloidal oder nanopartikulär verteilt vorliegt.

6. Feste pharmazeutische Zubereitung nach Anspruch 4 oder 5, **dadurch gekennzeichnet, daß** der Wirkstoff schwerlöslich ist.

7. Feste pharmazeutische Zubereitung nach einem der Ansprüche 4 bis 6, **dadurch gekennzeichnet, daß** sie neben dem Chitosanderivat einen weiteren polymeren Träger aufweist.

8. Verwendung einer pharmazeutischen Zubereitung, hergestellt nach einem Verfahren gemäß einem der vorangehenden Ansprüche, oder einer Zubereitung nach einem der Ansprüche 4 bis 6, zur Herstellung eines Arzneimittels.

9. Verwendung nach Anspruch 8 zur Herstellung eines Arzneimittels zur peroralen Applikation.

10. Verwendung nach Anspruch 8 oder 9 zur Herstellung eines Arzneimittels, weiches als Pulver, Granulat, Tablette oder Kapsel verabreicht wird.

11. Verwendung nach einem der Ansprüche 8 bis 10 zur Herstellung eines Arzneimittels, welches zum Zweck der Verabreichung in einer Flüssigkeit aufgelöst oder redispergiert wird.

12. Verwendung nach einem der Ansprüche 8 bis 11 zur Herstellung eines Arzneimittels mit kontrollierter Wirkstofffreisetzung.

13. Verwendung einer pharmazeutischen Zubereitung, hergestellt nach einem Verfahren gemäß einem der Ansprüche 1 bis 3 zur Herstellung eines Diagnostikums.

## Claims

1. Process for the production of a solid pharmaceutical preparation comprising at least one at least partially charged active substance, which active substance is present in the form of a nanosol in which the active substance is bonded to an oppositely charged chitosan derivative, said process being **characterized in that**
- an aqueous sol is prepared from a negatively charged or zwitterionic, acidic chitosan derivative and an active substance having positive charge by adding the active substance prior to or after dissolving the chitosan derivative or by uniting a solution of the chitosan derivative and an independently prepared solution of the active substance, the chitosan derivative being selected according to the type and relative number of its charged groups and in coordination with the type and relative number of the positively charged groups of the active substance such that at a certain pH value an isoionic state or charge equalization between active substance and carrier is achieved in the preparation,
- the pH value of the aqueous sol is adjusted such that an isoionic state results, possibly with colloidal or nano-scale active substance particles precipitating, and
- the thus-adjusted aqueous sol is dried.

2. Process according to any one of the preceding claims, **characterized in that** a poorly soluble active substance is used.

3. Process according to any one of the preceding claims, **characterized in that** apart from the chitosan derivative a further polymeric carrier substance is used.

4. Solid pharmaceutical preparation comprising at least one at least partially charged active substance, **characterized in that** the active substance is present in the form of a nanosol, said active substance having a positive charge and being bonded to a negatively charged or zwitterionic, acidic chitosan derivative.

5. Solid pharmaceutical preparation according to claim 4, **characterized in that** the active substance is present in the nanosol in colloidal or in nanoparticulate form.

6. Solid pharmaceutical preparation according to claim 4 or 5, **characterized in that** the active substance is poorly soluble.

7. Solid pharmaceutical preparation according to any one of claims 4 to 6, **characterized in that** it contains a further polymeric carrier apart from the chitosan derivative.

8. Use of a pharmaceutical preparation prepared according to a process according to any one of the preceding claims, or of a preparation according to any one of claims 4 to 6, for the production of a medicinal product.

9. Use according to Claim 8 for the production of a medicinal product for peroral application.

10. Use according to Claim 8 or 9 for the production of a medicinal product which is administered as a powder, granulate, tablet or capsule.

11. Use according to any one of Claims 8 to 10 for the production of a medicinal product which, for the purpose of administration, is dissolved or redispersed in a liquid.

12. Use according to any one of Claims 8 to 11 for the production of a medicinal product having controlled active substance release.

13. Use of a pharmaceutical preparation prepared according to a process according to any one of Claims 1 to 3 for the production of a diagnostic agent.

## Revendications

1. Procédé de fabrication d'une préparation pharmaceutique solide avec au moins une substance active au moins partiellement chargée, qui se présente sous forme d'un nanosol, dans lequel la substance active est liée à un dérivé de chitosan, chargé en sens inverse, **caractérisé en ce que**,
- un sol aqueux est fabriqué à partir d'un dérivé de chitosan acide chargé négativement ou zwitterionique et d'une substance active présentant une charge positive, dans lequel la substance active est ajoutée avant ou après la dissolution du dérivé de chitosan où une solution du dérivé de chitosan et une solution de la substance active fabriquée indépendamment, sont réunies mutuellement, le dérivé de chitosan étant choisi selon le type et le nombre relatif de ses groupes chargés, et en accord avec le type et le nombre relatif de groupes chargés positivement de la substance active, **en ce que**, pour une valeur de pH déterminée dans la préparation, un état isoionique ou une compensation de charge sont obtenus entre la substance active et le véhicule,
- la valeur de pH du sol aqueux est ajustée de sorte à former un état isoionique, des particules de substance active colloïdales ou à échelle nanométrique pouvant être produites, et
- le sol aqueux ajusté de cette façon est séché.

2. Procédé selon l'une quelconque des revendications précédentes, **caractérisé en ce qu'**une substance active peu soluble est employée.

3. Procédé selon l'une quelconque des revendications précédentes, **caractérisé en ce que**, outre le dérivé de chitosan, un véhicule polymère supplémentaire est employé.

4. Préparation pharmaceutique solide avec au moins une substance active partiellement chargée, **caractérisée en ce que** la substance active se présente sous la forme d'un nanosol, la substance active présentant une charge positive et étant liée à un dérivé de chitosan acide chargé négativement ou zwitterionique.

5. Préparation pharmaceutique solide selon la revendication 4, **caractérisée en ce que** la substance active se présente distribuée de façon colloïdale ou nanoparticulaire dans le nanosol.

6. Préparation pharmaceutique solide selon la revendication 4 ou 5, **caractérisée en ce que** la substance active est peu soluble.

7. Préparation pharmaceutique solide selon l'une quelconque des revendications 4 à 6, **caractérisée en qu'**elle présente, outre le dérivé de chitosan, un véhicule polymère supplémentaire.

8. Utilisation d'une préparation pharmaceutique, fabriquée d'après un procédé selon l'une quelconque des revendications précédentes, ou d'une préparation selon l'une quelconque des revendications 4 à 6, pour fabriquer un médicament.

9. Utilisation selon la revendication 8, pour la fabrication d'un médicament destiné à une application perorate.

10. Utilisation selon la revendication 8 ou 9, pour fabriquer un médicament qui est administré sous forme de poudre, de granulé, de comprimé ou de capsule.

11. Utilisation selon l'une quelconque des revendications 8 à 10, pour fabriquer un médicament qui est dissous ou re-dispersé dans un liquide dans le but d'être administré.

12. Utilisation selon l'une quelconque des revendications 8 à 11, pour fabriquer un médicament avec une libération contrôlée de la substance active.

13. Utilisation d'une préparation pharmaceutique, fabriquée d'après un procédé selon l'une quelconque des revendications 1 à 3, pour fabriquer une substance servant au diagnostic.
